# EUROPEAN PATENT APPLICATION

(11) **EP 4 692 299 A1**
(43) Date of publication of application: **11.02.2026**
(21) Application number: 24779704.6
(22) Date of filing: 19.03.2024
(51) Int. Cl.: C12M 1/00, C12Q 1/6844

(54) **NUCLEIC ACID ADSORPTION INHIBITOR, NUCLEIC ACID SOLUTION, AND NUCLEIC ACID AMPLIFICATION METHOD**

(30) Priority: 30.03.2023 JP 2023055743
(71) Applicant: NOF Corporation, Shibuya-ku Tokyo 150-6019 (JP)
(72) Inventor: SUZUKI, Hirotaka, Kawasaki-shi, Kanagawa 210-0865 (JP); MATSUDA, Masaru, Kawasaki-shi, Kanagawa 210-0865 (JP)
(74) Representative: J A Kemp LLP
(86) International application number: PCT/JP2024/010671
(87) International publication number: WO 2024/203579

(57) **Abstract**

The present invention provides a nucleic acid adsorption inhibitor containing one or more polymers selected from the following Group A :
[Group A]
polymer A1: a polymer consisting only of constitutional units derived from monomer a having a phosphoryl choline group;
polymer A2: a copolymer comprising constitutional units derived from the aforementioned monomer a, and constitutional units derived from monomer b having a carboxy group; and
polymer A3: a copolymer comprising constitutional units derived from the aforementioned monomer a, and constitutional units derived from monomer c having an (alkoxy having 1 to 20 carbon atoms)-carbonyl group;
provided that polymer A1 to polymer A3 do not contain a constitutional unit containing a cationic functional group other than a phosphorylcholine group.

## Description

### [Technical Field]

The present invention relates to a nucleic acid adsorption inhibitor, a nucleic acid solution, and a nucleic acid amplification method.

### [Background Art]

DNA (deoxyribonucleic acid) is one type of nucleic acid and a biopolymer that serves as a carrier of genetic information in many organisms on the earth.

Examples of methods for detecting, quantifying, and sequencing DNA include methods using DNA detection techniques such as agarose gel electrophoresis, nucleic acid amplification techniques represented by PCR method and LAMP method, nucleic acid quantification techniques applying these techniques, such as quantitative PCR and digital PCR, sequencing techniques such as the Sanger method and next-generation sequencing, or techniques that perform these on microarrays and biochips.

However, in all of the above-mentioned applications, nonspecific adsorption of DNA often poses a problem. That is, when manipulating, storing, or transporting DNA, DNA adsorbs to the surface of containers, microchips, and the like, particularly the surface of plastic members, and often causes a decrease in the concentration of free DNA.

In particular, when performing DNA detection, quantification, and base sequence analysis, target DNA at extremely low concentrations ranging from micromolar to attomole or less is analyzed. Furthermore, it is often difficult to obtain excess DNA, where DNA loss due to adsorption to the inner walls of containers causes a serious problem.

Problems similar to the above also occur with nucleic acids other than double-stranded DNA.

Against this background, various plastic modification technologies have been investigated to inhibit adsorption of nucleic acids to plastic containers. For example, Non Patent Literature 1 introduces a plastic tube product with a specific modification, and describes that use of this product in preparing and storing the nucleic acid concentration standards results in inhibition of nucleic acid adsorption to the container, improvement of nucleic acid yield, and improvement of the accuracy and sensitivity of applications that use the standards.

In addition, Patent Literatures 1 and 2 disclose technologies for suppressing nucleic acid adsorption by coating the surfaces of containers or biochips with a specific coating material, and by forming a layer containing a specific polymeric substance.

### [Citation List]

### [Patent Literature]

[Patent Literature 1]
   JP 2012-78365 A
[Patent Literature 2]
   JP 2006-258630 A

### [Non Patent Literature]

[Non Patent Literature 1]
Eppendorf PCR Consumables - Compatibility Guide for PCR and qPCR Cyclers

### [Summary of Invention]

### [Technical Problem]

However, the approach of improving plastics, as in Non Patent Literature 1, requires previous replacement of all members that will come into contact with nucleic acids with members that have been modified with the technology, which is inconvenient. Similarly, the techniques described in Patent Literatures 1 and 2 also require previous application of the technology to all members that will come into contact with nucleic acids, which is inconvenient.

In view of the above-mentioned problem, the present invention aims to provide a technology that achieves inhibition of nucleic acid adsorption by an approach that improves the nucleic acid solution side. To be specific, the present invention aims to provide a nucleic acid adsorption inhibitor that, when added to a nucleic acid solution, can inhibit the adsorption of the nucleic acid to containers and members, a nucleic acid solution containing the same, and a nucleic acid amplification method using the nucleic acid solution. This approach is also advantageous in that the addition concentration can be controlled according to the property of samples, which allows for a high degree of flexibility.

### [Solution to Problem]

[1] A nucleic acid adsorption inhibitor comprising one or more polymers selected from the following Group A
   [Group A]
   polymer A1: a polymer consisting only of constitutional units derived from monomer a having a phosphoryl choline group;
   polymer A2: a copolymer comprising constitutional units derived from the aforementioned monomer a, and constitutional units derived from monomer b having a carboxy group; and
   polymer A3: a copolymer comprising constitutional units derived from the aforementioned monomer a, and constitutional units derived from monomer c having an (alkoxy having 1 to 20 carbon atoms)-carbonyl group;
   provided that polymer A1 to polymer A3 do not contain a constitutional unit containing a cationic functional group other than a phosphorylcholine group.
[2] The nucleic acid adsorption inhibitor of the aforementioned [1], wherein the aforementioned monomer a is at least one selected from the group consisting of 2-methacryloyloxyethyl phosphorylcholine and 2-methacrylamidoethyl phosphorylcholine, preferably 2-methacryloyloxyethyl phosphorylcholine..
[3] The nucleic acid adsorption inhibitor of the aforementioned [1] or [2], wherein the aforementioned monomer b is at least one selected from the group consisting of (meth)acrylic acid and crotonic acid, preferably (meth)acrylic acid, more preferably methacrylic acid.
[4] The nucleic acid adsorption inhibitor of any one of the aforementioned [1] to [3], wherein the aforementioned monomer c is an (alkyl having 1 to 20 carbon atoms) ester of methacrylic acid, preferably an with an (alkyl having 1 to 6 carbon atoms) ester of methacrylic acid, further preferably butyl methacrylate.
[5] The nucleic acid adsorption inhibitor of any one of the aforementioned [1] to [4], wherein the aforementioned polymer A1 has a weight average molecular weight of 10,000 to 2,000,000, preferably 100,000 to 2,000,000, more preferably 500,000 to 1,500,000.
[6] The nucleic acid adsorption inhibitor of any one of the aforementioned [1] to [5], wherein the aforementioned polymer A2 has a weight average molecular weight of 50,000 to 2,000,000, preferably 100,000 to 2,000,000, more preferably 300,000 to 1,500,000, further preferably 300,000 to 1,000,000.
[7] The nucleic acid adsorption inhibitor of any one of the aforementioned [1] to [6], wherein the aforementioned polymer A3 has a weight average molecular weight of 5,000 to 200,000, preferably 10,000 to 200,000, more preferably 10,000 to 50,000, further preferably 30,000 to 50,000.
[8] The nucleic acid adsorption inhibitor of any one of the aforementioned [1] to [7], wherein a proportion of the constitutional unit derived from the aforementioned monomer a is 1 to 99 mol%, preferably 5 to 90 mol%, more preferably 10 to 60 mol%, further preferably 20 to 40 mol%, relative to the total constitutional units of the aforementioned polymer A2.
[9] The nucleic acid adsorption inhibitor of any one of the aforementioned [1] to [8], wherein a proportion of the constitutional unit derived from the aforementioned monomer b is 1 to 99 mol%, preferably 10 to 95 mol%, more preferably 40 to 90 mol%, further preferably 60 to 80 mol%, relative to the total constitutional units of the aforementioned polymer A2.
[10] The nucleic acid adsorption inhibitor of any one of the aforementioned [1] to [9], wherein the aforementioned polymer A2 is a copolymer consisting only of constitutional units derived from the aforementioned monomer a and constitutional units derived from the aforementioned monomer b.
[11] The nucleic acid adsorption inhibitor of any one of the aforementioned [1] to [10], wherein a proportion of the constitutional unit derived from the aforementioned monomer a is 10 to 90 mol%, preferably 20 to 70 mol%, more preferably 25 to 50 mol%, relative to the total constitutional units of the aforementioned polymer A3.
[12] The nucleic acid adsorption inhibitor of any one of the aforementioned [1] to [11], wherein a proportion of the constitutional unit derived from the aforementioned monomer c is 10 to 90 mol%, preferably 30 to 80 mol%, more preferably 50 to 75 mol%, relative to the total constitutional units of the aforementioned polymer A3.
[13] The nucleic acid adsorption inhibitor of any one of the aforementioned [1] to [12], wherein the aforementioned polymer A3 is a copolymer consisting only of constitutional units derived from the aforementioned monomer a and constitutional units derived from the aforementioned monomer c.
[14] The nucleic acid adsorption inhibitor of any one of the aforementioned [1] to [13], wherein the aforementioned nucleic acid is a double-stranded nucleic acid, preferably a double-stranded DNA.
[15] A nucleic acid solution comprising the nucleic acid adsorption inhibitor of any one of the aforementioned [1] to [13], and a nucleic acid.
[16] The nucleic acid solution of the aforementioned [15], which is a nucleic acid aqueous solution.
[17] The nucleic acid solution of the aforementioned [15] or [16], wherein the aforementioned nucleic acid is a double-stranded nucleic acid, preferably a double-stranded DNA.
[18] A method for amplifying a nucleic acid, comprising using the nucleic acid solution of any one of the aforementioned [15] to [17].
[19] A method for inhibiting adsorption of a nucleic acid in a nucleic acid solution to a member in contact with the aforementioned nucleic acid solution, comprising mixing the nucleic acid adsorption inhibitor of any one of the aforementioned [1] to [13], the aforementioned nucleic acid, and a solvent.
[20] The method of the aforementioned [17], wherein the aforementioned nucleic acid solution is a nucleic acid aqueous solution, and the aforementioned solvent is water.
[21] The method of the aforementioned [19] or [20], wherein the aforementioned nucleic acid is a double-stranded nucleic acid, preferably a double-stranded DNA.

### [Advantageous Effects of Invention]

The nucleic acid adsorption inhibitor of the present invention can inhibit free nucleic acids present in the solution to which it is added from adsorbing to the surfaces of containers, microchips, and the like. Therefore, in various tests using nucleic acid samples as specimens, for example, improved detection sensitivity and improved storage stability of nucleic acid-containing specimens and standards can be expected.

### [Description of Embodiments]

The present invention is described in detail below. Respective descriptions in the present specification can be combined with each other, unless it is clear that they cannot be combined.

When stepwise numerical ranges are described in the present specification, the lower limit and the upper limit of each numerical range can be combined. In the case of, for example, "preferably 10 to 100, more preferably 20 to 90", the preferred lower limit 10 can be combined with the more preferred upper limit 90 (i.e., the numerical range of "10 to 90" is also within the range of the present specification).

### [Nucleic acid adsorption inhibitor]

In the present invention, the "nucleic acid adsorption inhibitor" refers to an agent to be added to a nucleic acid solution to inhibit adsorption of nucleic acids to the surface of members that come into contact with the solution in containers, microchips, and the like that handle the nucleic acid solution.

The nucleic acid adsorption inhibitor of the present invention contains one or more polymers selected from the following Group A:
[Group A]
polymer A1: a polymer consisting only of constitutional units derived from monomer a having a phosphoryl choline group;
polymer A2: a copolymer comprising constitutional units derived from the aforementioned monomer a, and constitutional units derived from monomer b having a carboxy group; and
polymer A3: a copolymer comprising constitutional units derived from the aforementioned monomer a, and constitutional units derived from monomer c having an (alkoxy having 1 to 20 carbon atoms)-carbonyl group;
provided that polymer A1 to polymer A3 do not contain constitutional units containing a cationic functional group other than a phosphorylcholine group.

In the present specification, the "constitutional units" mean repeat units derived from monomers in polymers. Therefore, the "constitutional unit" does not include structures not repeated in the polymer (e.g., structure derived from polymerization initiator and the like).

In the present specification, the "phosphorylcholine group" means a monovalent group represented by the following formula (in the following formula, * indicates a bonding position).

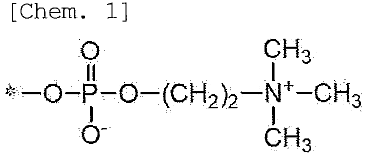

In the present specification, the "(alkoxy having 1 to 20 carbon atoms)-carbonyl group" refers to a group in which an alkoxy group having 1 to 20 carbon atoms is bonded to a carbonyl (-CO-). Examples include acetyl group, propionyl group, butanoyl group, pentanoyl group, hexanoyl group, heptanoyl group, octanoyl group, nonanoyl group, decanoyl group, undecanoyl group, dodecanoyl group, tridecanoyl group, hexadecanoyl group, pentadecanoyl group, octadecanoyl group, nonadecanoyl group, icosanoyl group, and the like.

Examples of monomer a for the constitutional units derived from monomer a contained in polymers A1 to A3 are not particularly limited as long as they contain a phosphorylcholine group and are polymerizable with monomer b and monomer c. Preferred examples include compounds containing a phosphorylcholine group and a vinyl group, such as 2-(meth)acryloyloxyethyl phosphorylcholine, 2-(meth)acrylamidoethyl phosphorylcholine, alkylphosphorylcholine allyl ether, and alkylphosphorylcholine vinyl ether.

As used herein, the "(meth)acryloyloxy group" basically means an acryloyloxy group or a methacryloyloxy group. When a plurality of the (meth)acryloyloxy group may be present, the "(meth)acryloyloxy" means an acryloyloxy group and/or a methacryloyloxy group. The below-mentioned "(meth)acrylamide", "(meth)acrylic acid", and "(meth)acrylate" also mean the same as the "(meth)acryloyloxy group".

The monomer a is more preferably 2-methacryloyloxyethyl phosphorylcholine or 2-methacrylamidoethyl phosphorylcholine, further preferably 2-methacryloyloxyethyl phosphorylcholine, from the aspects of storage stability of the polymer and availability of raw materials.

Only one kind of monomer a may be used, or two or more kinds thereof may be used in combination. As monomer a, a commercially available product can be used.

Polymer A1 is a polymer consisting only of constitutional units derived from monomer a. In the present specification, "polymer consisting only of constitutional units derived from monomer a" refers to a polymer whose total constitutional units consists only of constitutional units derived from monomer a, and the "constitutional units" mean repeat units derived from monomers in polymers. Therefore, the "constitutional unit" does not include structures not repeated in the polymer (e.g., structure derived from polymerization initiator and the like). Polymer A1 may be a homopolymer consisting only of constitutional units derived from one kind of monomer a, or a copolymer consisting only of constitutional units derived from two or more kinds of monomer a.

While the weight average molecular weight of polymer A1 is not particularly limited, it is preferably 10,000 to 2,000,000, more preferably 100,000 to 2,000,000, further preferably 500,000 to 1,500,000, from the aspect of the nucleic acid adsorption inhibitory effect.

In the present specification, the weight average molecular weight can be determined based on polyethylene glycol by gel filtration chromatography using, for example, EcoSEC system (manufactured by Tosoh Corporation) or the like.

Examples of monomer b for the constitutional units derived from monomer b contained in polymer A2 are not particularly limited as long as they contain a carboxy group and are polymerizable with monomer a. For example, compounds containing a carboxy group and a vinyl group such as (meth)acrylic acid, crotonic acid, 3-methylcrotonic acid, angelic acid, tiglic acid, fumaric acid, maleic acid, itaconic acid, citraconic acid and the like can be mentioned. As monomer b, (meth)acrylic acid or crotonic acid is preferred, (meth)acrylic acid is more preferred, and methacrylic acid is further preferred, from the aspect of the ease of polymerization reactions.

Only one kind of monomer b may be used, or two or more kinds thereof may be used in combination. As monomer b, a commercially available product can be used.

From the aspect of the nucleic acid adsorption inhibitory effect, the proportion of constitutional units derived from monomer b relative to the total constitutional units of polymer A2 is preferably 1 to 99 mol%, more preferably 10 to 95 mol%, further preferably 40 to 90 mol%, further more preferably 60 to 80 mol%.

From the aspect of the nucleic acid adsorption inhibitory effect, the proportion of constitutional units derived from monomer a relative to the total constitutional units of polymer A2 is preferably 1 to 99 mol%, more preferably 5 to 90 mol%, further preferably 10 to 60 mol%, further more preferably 20 to 40 mol%. Polymer A2 is particularly preferably a copolymer consisting only of constitutional units derived from monomer a and constitutional units derived from monomer b. In the present specification, the "copolymer consisting only of constitutional units derived from monomer a and constitutional units derived from monomer b" means a copolymer whose total constitutional units consist only of constitutional units derived from monomer a and constitutional units derived from monomer b.

While the weight-average molecular weight of polymer A2 is not particularly limited, from the aspect of the nucleic acid adsorption inhibitory effect, it is preferably 50,000 to 2,000,000, more preferably 100,000 to 2,000,000, further preferably 300,000 to 1,500,000, further more preferably 300,000 to 1,000,000.

Examples of monomer c for the constitutional units derived from monomer c contained in polymer A3 are not particularly limited as long as they contain an (alkoxy having 1 to 20 carbon atoms)-carbonyl group and are polymerizable with monomer a. Examples include, but are not limited to, (alkyl having 1 to 20 carbon atoms) esters of compounds containing a carboxy group and a vinyl group, such as (alkyl having 1 to 20 carbon atoms) ester of (meth)acrylic acid (e.g., methyl (meth)acrylate, ethyl (meth)acrylate, propyl (meth)acrylate, butyl (meth)acrylate, pentyl (meth)acrylate, hexyl (meth)acrylate, heptyl (meth)acrylate, octyl (meth)acrylate, nonyl (meth)acrylate, decyl (meth)acrylate, undecyl (meth)acrylate, lauryl (meth)acrylate, tridecyl (meth)acrylate, myristyl (meth)acrylate, pentadecyl (meth)acrylate, cetyl (meth)acrylate, margaryl (meth)acrylate, stearyl (meth)acrylate, nonadecyl (meth)acrylate, arachidyl (meth)acrylate, and the like); (alkyl having 1 to 20 carbon atoms) ester of crotonic acid; (alkyl having 1 to 20 carbon atoms) ester of 3-methylcrotonic acid; (alkyl having 1 to 20 carbon atoms) ester of angelic acid; (alkyl having 1 to 20 carbon atoms) ester of tiglic acid; (alkyl having 1 to 20 carbon atoms) ester of fumaric acid; (alkyl having 1 to 20 carbon atoms) ester of maleic acid; (alkyl having 1 to 20 carbon atoms) ester of itaconic acid; and (alkyl having 1 to 20 carbon atoms) ester of citraconic acid. Among these, from the aspect of the storage stability of the polymer, (alkyl having 1 to 20 carbon atoms) esters of methacrylic acid such as methyl methacrylate, ethyl methacrylate, propyl methacrylate, butyl methacrylate, pentyl methacrylate, hexyl methacrylate, heptyl methacrylate, octyl methacrylate, nonyl methacrylate, decyl methacrylate, undecyl methacrylate, lauryl methacrylate, tridecyl methacrylate, myristyl methacrylate, pentadecyl methacrylate, cetyl methacrylate, margaryl methacrylate, stearyl methacrylate, nonadecyl methacrylate, and arachidyl methacrylate are preferred; from the aspect of the nucleic acid adsorption inhibitory effect, (alkyl having 1 to 6 carbon atoms) esters of methacrylic acid such as methyl methacrylate, ethyl methacrylate, propyl methacrylate, butyl methacrylate, pentyl methacrylate, and hexyl methacrylate, are more preferred, and butyl methacrylate is even more preferred.

Only one kind of monomer c may be used, or two or more kinds thereof may be used in combination. As monomer c, a commercially available product can be used.

From the aspect of the nucleic acid adsorption inhibitory effect, the proportion of constitutional units derived from monomer c relative to the total constitutional units of polymer A3 is preferably 10 to 90 mol%, more preferably 30 to 80 mol%, further preferably 50 to 75 mol%.

From the aspect of the nucleic acid adsorption inhibitory effect, the proportion of constitutional units derived from monomer a relative to the total constitutional units of polymer A3 is preferably 10 to 90 mol%, more preferably 20 to 70 mol%, further preferably 25 to 50 mol%. It is particularly preferable that polymer A3 is a copolymer consisting only of constitutional units derived from monomer a and constitutional units derived from monomer c. In the present specification, the "copolymer consisting only of constitutional units derived from monomer a and constitutional units derived from monomer c" means a copolymer whose total constitutional units consist only of constitutional units derived from monomer a and constitutional units derived from monomer c.

While the weight average molecular weight of polymer A3 is not particularly limited, it is preferably 5,000 to 200,000, more preferably 10,000 to 200,000, further preferably 10,000 to 50,000, further more preferably 30,000 to 50,000, from the aspect of the nucleic acid adsorption inhibitory effect.

Polymers A2 and A3 may contain, as long as the effect of the present invention is not impaired, constitutional units derived from a monomer other than the aforementioned monomer (hereinafter to be indicated as "other monomer"). However, other monomers contain only anionic, amphoteric, or neutral functional groups.

Examples of other monomer include glycerol mono(meth)acrylate, benzyl (meth)acrylate, isobornyl (meth)acrylate, and the like. The proportion of constitutional units derived from other monomer relative to the total constitutional units of polymers A2 and A3, respectively, is preferably not more than 40 mol%, more preferably not more than 20 mol%, further preferably not more than 10 mol%. Further more preferably, polymers A2 and A3 do not contain constitutional units derived from other monomer.

When the aforementioned polymer is a copolymer, the copolymer may be a random copolymer, an alternating copolymer, a block copolymer, a graft polymer, or a copolymer containing two or more structures thereof. It is preferably a random copolymer from the aspect of the manufacturability of the polymer.

As each monomer for the preparation of the polymers A1 to A3, a commercially available product may be used, or the monomer may be produced by a known method. The polymers A1 to A3 can be produced by known methods (e.g., the method described in WO 2018/216628).

The nucleic acid adsorption inhibitor of the present invention can contain components other than polymers A1 to A3, as long as the nucleic acid adsorption inhibitory effect of the polymers A1 to A3 is not impaired. Such other components are not particularly limited, and may be appropriately selected from, for example, those exemplified as "other components" in the nucleic acid solution of the present invention described below.

Polymers A1 to A3 contained in a nucleic acid solution can be easily used as a nucleic acid adsorption inhibitor.

Methods for containing the nucleic acid adsorption inhibitor of the present invention in a nucleic acid solution include adding polymers A1 to A3 to an already prepared nucleic acid solution and dissolving them; dissolving the nucleic acid adsorption inhibitor of the present invention in advance in a solvent such as a buffer that dissolves nucleic acids; or placing the nucleic acid adsorption inhibitor of the present invention in advance in a container in which the nucleic acid solution will be prepared (e.g., coating the inner surface of the container with the nucleic acid adsorption inhibitor of the present invention) and then adding the nucleic acid solution to dissolve therein.

The material of the containers, members, and the like to which adsorption of nucleic acid is prevented using the nucleic acid adsorption inhibitor of the present invention is preferably resin, more preferably polypropylene.

The final concentration of the nucleic acid adsorption inhibitor of the present invention to be added to the aforementioned nucleic acid solution is preferably 0.01 to 5 w/v%, more preferably 0.1 to 1 w/v%, further preferably 0.1 to 0.5 w/v%. When the amount added is too small, the nucleic acid adsorption inhibitory effect may not be achieved, whereas when the amount added is too large, problems such as reaction inhibition may occur when the nucleic acid solution is used in applications such as enzyme reactions

The type of nucleic acid to which the nucleic acid adsorption inhibitor of the present invention can be applied is not particularly limited. It is preferably double-stranded nucleic acid (e.g., double-stranded DNA, double-stranded RNA, DNA:RNA hybrid), more preferably double-stranded DNA.

Here, the nucleic acid may be artificially synthesized by chemical synthesis, in vitro synthesis (e.g., reverse transcription reaction), PCR and the like, or may be prepared from cells, microorganisms, viruses, or the like by known methods. Here, the cells, microorganisms, viruses, and the like may be those collected from human, animals, or plants in the natural world or environment, or may be those obtained by isolation and culture.

### [Nucleic acid solution]

The present invention further provides a nucleic acid aqueous solution containing the nucleic acid adsorption inhibitor of the present invention and nucleic acid.

The concentration of the nucleic acid adsorption inhibitor in the nucleic acid solution of the present invention is, as described above, a final concentration of 0.01 to 5w/v%, more preferably 0.1 to 1w/v%, further preferably 0.1 to 0.5w/v%.

The nucleic acid contained in the nucleic acid solution of the present invention is preferably a nucleic acid in which two or more molecular chains are associated, more preferably double-stranded nucleic acid, further preferably double-stranded DNA.

The nucleic acid may be artificially synthesized by chemical synthesis, in vitro synthesis (e.g., reverse transcription reaction), PCR and the like, or may be provided as a virus, bacterial cell, cell, body fluid, tissue, etc., or a suspension of any of these, or a nucleic acid extract prepared from any of these. The virus, bacterial cell, cell, body fluid, tissue, etc. may be collected from human, animals, or plants in the natural world or environment, or may be obtained by isolation and culture.

The concentration of the nucleic acid is determined appropriately according to the application using the nucleic acid.

The nucleic acid solution of the present invention may contain other components as long as the effects of the present invention are not impaired. Examples of other components include polyol, polyether, protein, salts, buffer, surfactant, solvent, biochemical reagent, dyes, preservative, oil, solid support, and the like.

Examples of polyol include glycerol, sucrose, glucose, and the like.

Examples of polyether include polyoxyethylene glycol, and the like.

Examples of protein include albumin, gelatin, casein, enzyme, and the like.

Examples of the salts include amino acid salts, peptide salts, alkali metal salts, alkaline earth metal salts, and salts of organic acids such as ethylenediaminetetraacetic acid.

Examples of the buffer include Tris-HCl buffer, Good's buffer, glycine buffer, borate buffer, TE buffer, TAE buffer, TBE buffer, SSC buffer, and the like.

Examples of the surfactant include polyoxyethylene alkyl ether, polyoxyethylene sorbitan monoalkyl ether, alkyl betaine, and the like.

Examples of the solvent include water and organic solvents. Examples of the organic solvent include ethanol, propanol, isoamyl alcohol, glycerin, dimethylformamide, dimethyl sulfoxide, tetrahydrofuran, chloroform, and phenol. The solvent is preferably water, and the nucleic acid solution of the present invention is preferably an aqueous nucleic acid solution.

Examples of the biochemical reagent include flavins.

Examples of the dyes include nucleic acid staining reagents such as ethidium bromide and SYBR^{™} Green I, fluorescent dyes such as ROX^{™} Dye, and colorants such as Orange G, bromophenol blue, and xylene cyanol FF.

Examples of the preservative include sodium azide, parahydroxybenzoic acid preparation, dehydroacetic acid preparation, and Proclin preparation.

Examples of the oil include mineral oil.

Examples of the solid support include silica beads and magnetic beads.

### [Nucleic acid amplification method]

The nucleic acid solution of the present invention can be subjected to an enzyme reaction while still containing the nucleic acid adsorption inhibitor of the present invention.

Examples of the enzyme reaction include cleavage by nuclease, reverse transcription by reverse transcriptase, replication by DNA polymerase, nucleic acid amplification applying these, and the like. The enzyme reaction in the present invention is preferably replication of DNA or RNA, more preferably PCR.

### [Method for inhibiting adsorption of nucleic acid]

The present invention provides a method for inhibiting adsorption of nucleic acids in a nucleic acid solution to a member in contact with the aforementioned nucleic acid solution, including mixing the nucleic acid adsorption inhibitor of the present invention, the aforementioned nucleic acid, and a solvent. The aforementioned mixing is not particularly limited. For example, (1) the nucleic acid adsorption inhibitor of the present invention, a nucleic acid, and a solvent may be mixed; (2) a solution containing a nucleic acid and a solvent, and the nucleic acid adsorption inhibitor of the present invention may be mixed; or (3) a solution containing the nucleic acid adsorption inhibitor of the present invention and a solvent, and the nucleic acid adsorption inhibitor may be mixed. The explanations of the nucleic acid solution obtained by mixing the nucleic acid adsorption inhibitor of the present invention, a nucleic acid, and a solvent (e.g., the kind of nucleic acid used, the concentration of the nucleic acid adsorption inhibitor of the present invention, and other components) are the same as those of the aforementioned nucleic acid solution of the present invention.

### [Example]

The present invention is explained in more detail in the following by referring to Examples and the like, which are not to be construed as limitative.

### [Synthesis of polymer]

Polymer A1-1, polymer A2-1, polymer A3-1 to polymer A3-3, which are within the scope of the present invention, and polymer Z-1, which is outside the scope of the present invention, were prepared as follows. The obtained polymers were dissolved in Water, Nuclease free (manufactured by Nippon Gene Co., Ltd., hereafter referred to as "PW") to a concentration 10 times the final concentration described for each condition in the Examples and Comparative Examples described below, and the obtained polymer aqueous solutions were used.

The six kinds of polymers prepared are summarized in Table 1.

### [Synthetic Example 1]

Polymer A1-1 corresponding to polymer A1 was prepared as follows.

2-Methacryloyloxyethyl phosphorylcholine (hereinafter to be referred to as "MPC") (100 g) was weighed into a glass flask for polymerization and purified water (150 g) was added to dissolve same. To the obtained solution was added 4,4'-azobis(4-cyanovaleric acid) (hereinafter to be referred to as ACVA) (2.0 g). The solution was heated to 70°C and stirred under a nitrogen atmosphere for 6 hr to perform polymerization. The obtained polymerization solution was purified by dialysis using a semipermeable membrane with a molecular cutoff of 20,000, and then freeze-dried to obtain polymer A1-1. The weight average molecular weight of polymer A1-1 was 1,030,000 based on polyethylene glycol as measured by GPC under the below-mentioned conditions.

### [Synthetic Example 2]

Polymer A2-1 corresponding to polymer A2 was prepared as follows.

MPC (60 g) and methacrylic acid (hereinafter to be referred to as "MAc") (40 g) (MPC/MAc=30/70 (molar ratio)) were weighed into a glass flask for polymerization and purified water (567 g) was added to dissolve them. To the obtained solution was added ACVA (5.2 g). The solution was heated to 70°C and stirred under a nitrogen atmosphere for 6 hr to perform polymerization. The obtained polymerization solution was purified by dialysis using a semipermeable membrane with a molecular cutoff of 20,000, and then freeze-dried to obtain polymer A2-1. The weight average molecular weight of polymer A2-1 was 370,000 based on polyethylene glycol as measured by GPC under the below-mentioned conditions.

### [Synthetic Example 3]

Polymer A3-1 corresponding to polymer A3 was prepared as follows.

MPC (47 g) and butyl methacrylate (hereinafter to be referred to as "BMA") (53 g) (MPC/BMA=30/70 (molar ratio)) were weighed into a glass flask for polymerization and ethanol (hereinafter to be referred to as "EtOH") (900 g) was added to dissolve them and the solution was heated to 60°C. Under a nitrogen atmosphere, to the obtained solution was added 2,2'-azobis(isobutyronitrile) (hereinafter to be referred to as "AIBN") (2.4 g) and the mixture was stirred for 7 hr to perform polymerization. The obtained polymerization solution was purified by dialysis using a semipermeable membrane with a molecular cutoff of 3,000, and then freeze-dried to obtain polymer A3-1. The weight average molecular weight of polymer A3-1 was 90,000 based on polyethylene glycol as measured by GPC under the below-mentioned conditions.

### [Synthetic Example 4]

Polymer A3-2 corresponding to polymer A3 was prepared as follows.

MPC (78 g) and stearyl methacrylate (hereinafter to be referred to as "SMA") (22 g) (MPC/SMA=80/20) were weighed into a glass flask for polymerization and EtOH (667 g) was added to dissolve them, and the solution was heated to 60°C. Under a nitrogen atmosphere, to the obtained solution was added AIBN (3.3 g) and the mixture was stirred for 6 hr to perform polymerization. The obtained polymerization solution was purified by dialysis using a semipermeable membrane with a molecular cutoff of 3,000, and then freeze-dried to obtain polymer A3-2. The weight average molecular weight of polymer A3-2 was 40,000 based on polyethylene glycol as measured by GPC under the below-mentioned conditions.

### [Synthetic Example 5]

Polymer A3-3 corresponding to polymer A3 was prepared as follows.

MPC (57 g), BMA (28 g), and glycerol monomethacrylate (hereinafter to be referred to as "GLM") (15 g) (MPC/BMA/GLM=40/40/20 (molar ratio)) were weighed into a glass flask for polymerization ethanol (409 g) and purified water (201 g) were added to dissolve them. To the obtained solution was added AIBN (1.9 g). The solution was heated to 60°C and stirred under a nitrogen atmosphere for 5 hr to perform polymerization. The obtained polymerization solution was purified by dialysis using a semipermeable membrane with a molecular cutoff of 3,000, and then freeze-dried to obtain polymer A3-3. The weight average molecular weight of polymer A3-3 was 20,000 based on polyethylene glycol as measured by GPC under the below-mentioned conditions.

### [Synthetic Example 6]

Polymer Z-1 which is outside the range of the present invention was prepared as follows.

MPC (74 g) and N,N,N-trimethyl-N-(2-hydroxy-3-methacryloyloxypropyl)-ammonium chloride (hereinafter to be referred to as "QA") (26 g) (MPC/QA=70/30 (molar ratio)) were weighed into a glass flask for polymerization and purified water (525 g) was added to dissolve them. To the obtained solution was added2,2'-azobis(2-methylpropionamide) dihydrochloride (0.9 g). The solution was heated to 70°C and stirred under a nitrogen atmosphere for 2 hr to perform polymerization. The obtained polymerization solution was purified by dialysis using a semipermeable membrane with a molecular cutoff of 20,000, and then freeze-dried to obtain copolymer Z-1. The weight average molecular weight of copolymer Z-1 was 40,000 based on polyethylene glycol as measured by GPC under the below-mentioned conditions.

### [GPC measurement]

GPC measurement of polymer A1-1, polymer A2-1, polymer A3-1 to polymer A3-3, and polymer Z-1 obtained in Synthetic Examples 1 to 6 was performed under the following conditions.
GPC system: EcoSEC system (manufactured by Tosoh Corporation)
column: Shodex OHpak SB-802.5HQ (manufactured by Showa Denko K.K.), and SB-806HQ (manufactured by Showa Denko K.K.) connected in tandem
eluent: 20 mM sodium phosphate buffer (pH 7.4)
detector: differential refractive index detector
molecular weight standard: EasiVial PEG/PEO (manufactured by Agilent Technologies)
flow rate: 0.5 mL/min
column temperature: 40°C
sample: obtained polymer diluted with eluent to final concentration of 0.1 wt%
injection volume: 100 µL

**[Table 1]**

| | monomer a | | monomer b | | monomer c | | other monomer | | weight average molecular weight [×10³] |
|---|---|---|---|---|---|---|---|---|---|
| | kind | proportion [mol%] | kind | proportion [mol%] | kind | proportion [mol%] | kind | proportion [mol%] | |
| polymer A1-1 | MPC | 100 | - | - | - | - | - | - | 1030 |
| polymer A2-1 | MPC | 30 | MAc | 70 | - | - | - | - | 370 |
| polymer A3-1 | MPC | 30 | - | - | BMA | 70 | - | - | 90 |
| polymer A3-2 | MPC | 80 | - | - | SMA | 20 | - | - | 40 |
| polymer A3-3 | MPC | 40 | - | - | BMA | 40 | GLM | 20 | 20 |
| polymer Z-1 | MPC | 70 | - | - | - | - | QA | 30 | 40 |

### [Experimental Example 1]

To a double-stranded DNA solution was added polymer A1-1, polymer A2-1, polymer A3-1 to polymer A3-3, or polymer Z-1 obtained in Synthetic Examples 1 to 6 and the mixture was placed in a propylene tube and left standing at room temperature. Then, it was developed by agarose gel electrophoresis. The quantitative value of double-stranded DNA was compared with the control to calculate the residual ratio of DNA in the solution. That is, the amount of DNA remaining without adsorbing to the inner surface of the propylene tube or the microchip that handled the solution was compared.
1) λ-HindIII digest (manufactured by Takara Bio Inc.) was used as DNA and added to the DNA solution described below to a final concentration of 10 ng/µL.
2) As the 10X buffer, 400 mM Tris-HCl buffer (containing 100 mM sodium chloride, 60 mM magnesium chloride, 10 mM calcium chloride, pH 7.9) was prepared.
3) Each DNA aqueous solution shown in Table 2 was prepared and dispensed into a 2 mL (free-standing) polypropylene screw-cap tube (manufactured by AS ONE, hereafter referred to as "PP tube").
4) The solution was left standing at room temperature overnight.
5) 50X TAE (manufactured by Nippon Gene Co., Ltd.) was diluted according to the manufacturer's instructions to prepare an electrophoresis buffer (1X TAE). 100 mL of 1X TAE was added to 1 g of Agarose S (manufactured by Nippon Gene Co., Ltd.), boiled to dissolve same, and solidified to prepare an electrophoresis gel (1% gel).
6) 1/6-fold amount of 6X Loading Buffer, Triple Dye (manufactured by Nippon Gene Co., Ltd.) was added to each of the liquids for internal use after 4) and mixed to prepare an electrophoresis sample.
7) The sample from 6) was developed by agarose gel electrophoresis. The electrophoresis buffer and gel from 4) were used, and 5 µL of sample was applied. Electrophoresis was performed at 100V for about 30 min.
8) The gel was stained by immersing same for about 15 min in Midori Green Advance (manufactured by Nihon Genetics Co., Ltd.) diluted 10,000-fold with 1X TAE, followed by destaining in pure water for about 15 min.
9) Images were taken using a FASdigi Compact (manufactured by Nihon Genetics Co., Ltd.) under BlueGreen LED illumination. Quantitative values for the 6.6 kbp band were calculated using ImageJ (National Institutes of Health, USA) and shown as relative values with the control as 100. The results are summarized in Table 2.

**[Table 2]**

| | | | control 1 | Example 1-1 | Example 1-2 | Example 1-3 | Example 1-4 | Example 1-5 | Comparative Example 1-1 |
|---|---|---|---|---|---|---|---|---|---|
| composition | 10X buffer | | 10 µL | | | | | | |
| | λ-*Hin*d III digest | | 2 µL (final concentration 10 ng/µL) | | | | | | |
| | polymer | kind | - | polymer A1-1 | polymer A2-1 | polymer A3-1 | polymer A3-2 | polymer A3-3 | polymer Z-1 |
| | | amount added (*1) | - | 2 µL | 2 µL | 2 µL | 2 µL | 2 µL | 2 µL |
| | PW | | rest | | | | | | |
| | total amount | | 100 µL | | | | | | |
| results | quantitative value (*2) | | 100 | 155 | 204 | 189 | 112 | 141 | 0 |

| | | | | | | | | | |
|---|---|---|---|---|---|---|---|---|---|
| *1 All copolymers were added to a final concentration of 0.1% (w/v). *2 Shown as relative value with the control as 100. | | | | | | | | | |

Example 1-1 to Example 1-3 are examples in which polymer A1-1 to polymer A3-1 were respectively used as nucleic acid adsorption inhibitors. From the results, it is clear that the use of the polymers of the present invention inhibits DNA adsorption to polypropylene tubes and microchips, thereby increasing the amount of remaining DNA.

Example 1-4 is an example in which Polymer A3-2 was used as the nucleic acid adsorption inhibitor. Polymer A3-2 is an example of a polymer in which the alkyl group in monomer c, containing an (alkoxy having 1 to 20 carbon atoms)-carbonyl group, in Polymer A3-1 was changed from butyl (carbon number 4) to stearyl (carbon number 18). It can be seen that the DNA adsorption inhibitory effect was also exhibited even in this case.

Example 1-5 is an example in which Polymer A3-3 was used as the nucleic acid adsorption inhibitor. Polymer A3-3 is an example of a polymer in which Polymer A3-1 further contains another monomer. It can be seen that the DNA adsorption inhibitory effect was also exhibited even in this case.

### [Experimental Example 2]

An experiment similar to Experimental Example 1 was performed using LABORAN screw tube bottle No. 2 instead of PP tube (manufactured by AS ONE, hereafter referred to as "glass tube").
1) Each DNA aqueous solution shown in Table 3 was prepared and dispensed into a glass tube.
2) Other conditions and procedures were the same as in Experimental Example 1. The results are shown in Table 3.

**[Table 3]**

| | | | control 2 | Example 2-1 | Example 2-2 | Example 2-3 | Comparative Example 2-1 |
|---|---|---|---|---|---|---|---|
| composition | 10X buffer | | 10 µL | | | | |
| | λ-*Hin*d III digest | | 2 µL (final concentration 10 ng/µL) | | | | |
| | polymer | kind | - | polymer A1-1 | polymer A2-1 | polymer A3-1 | polymer Z-1 |
| | | amount added (*3) | - | 2 µL | 2 µL | 2 µL | 2 µL |
| | PW | | rest | | | | |
| | total amount | | 100 µL | | | | |
| results | quantitative value (*4) | | 100 | 119 | 120 | 122 | 0 |

| | | | | | | | |
|---|---|---|---|---|---|---|---|
| *3 All copolymers were added to a final concentration of 0.1% (w/v). *4 Shown as relative value with the control as 100. | | | | | | | |

Example 2-1 to Example 2-3 and Comparative Example 2-1 are the results of experiments similar to Example 1-1 to Example 1-3, and Comparative Example 1-1, respectively, except that glass tubes were used instead of PP tubes. From the results, it is clear that the use of the polymers of the present invention inhibits DNA adsorption even when the container material is glass.

### [Industrial Applicability]

By simply adding the nucleic acid adsorption inhibitor of the present invention to a nucleic acid solution, the adsorption of the nucleic acid to members in contact with the aqueous solution can be inhibited in a container or the like. As a result, reduction of loss due to adsorption to containers and the like during nucleic acid extraction, namely, improvement of recovery rates, can be expected and improvement of detection sensitivity can be expected, in, for example, genetic testing in the medical, veterinary, and forensic fields. Furthermore, improvement of storage stability and transportation stability can be expected by inhibiting the reduction in effective concentration due to adsorption to containers and the like during storage and transportation of nucleic acid-containing specimens for genetic testing.

This application is based on a patent application No. 2023-055743 filed in Japan, the contents of which are encompassed in full herein.

## Claims

1. A nucleic acid adsorption inhibitor comprising one or more polymers selected from the following Group A
[Group A]
polymer A1: a polymer consisting only of constitutional units derived from monomer a having a phosphoryl choline group;
polymer A2: a copolymer comprising constitutional units derived from the monomer a, and constitutional units derived from monomer b having a carboxy group; and
polymer A3: a copolymer comprising constitutional units derived from the monomer a, and constitutional units derived from monomer c having an (alkoxy having 1 to 20 carbon atoms)-carbonyl group;
provided that polymer A1 to polymer A3 do not contain a constitutional unit containing a cationic functional group other than a phosphorylcholine group.

2. A nucleic acid solution comprising the nucleic acid adsorption inhibitor according to claim 1, and a nucleic acid.

3. A method for amplifying a nucleic acid, comprising using the nucleic acid solution according to claim 2.
